**Europäisches Patentamt**

(19) **European Patent Office**

**Office européen des brevets**

(11) Numéro de publication: **0 269 531**
**B1**

(12) # FASCICULE DE BREVET EUROPEEN

(45) Date de publication du fascicule du brevet:
**16.08.90**

(51) Int. Cl.⁵: **G01N 33/12**, G01N 27/04

(21) Numéro de dépôt: **87420296.3**

(22) Date de dépôt: **29.10.87**

(54) **Dispositif de détection de la nature d'un milieu de viande.**

(30) Priorité: **30.10.86 FR 8615301**

(43) Date de publication de la demande:
**01.06.88 Bulletin 88/22**

(45) Mention de la délivrance du brevet:
**16.08.90 Bulletin 90/33**

(84) Etats contractants désignés:
**AT BE CH DE ES GB GR IT LI LU NL SE**

(56) Documents cités:
**AU-B- 482 112**
**DE-A- 2 107 114**
**FR-A- 1 550 169**
**US-A- 3 224 320**
**US-A- 3 237 664**

(73) Titulaire: **UNION FINANCIERE POUR LE DEVELOPPEMENT DE L'ECONOMIE CEREALIERE-UNIGRAINS (Société Anonyme de droit français), 8, avenue du Président Wilson, F-75016 Paris(FR)**

(72) Inventeur: **Romand, Paul, La Batie Besayes, F-26300 Bourg de Péage(FR)**

(74) Mandataire: **Maureau, Philippe et al, Cabinet Germain & Maureau Le Britannia - Tour C 20, bld Eugène Déruelle Boîte Postale 3011, F-69392 Lyon Cédex 03(FR)**

ACTORUM AG

## Description

La présente invention a pour objet un dispositif destiné notamment à détecter la nature ("gras", "maigre"...) d'un milieu de viande ainsi que le passage d'un milieu à un autre et notamment celui d'un milieu gras à un milieu maigre dans un morceau de viande ou similaire, lors du découpage de celui-ci.

En effet, les pièces di viande sont, de plus en plus, découpées automatiquement. Or, étant donné les difféérences de coût très importantes entre le "gras" et le "maigre" d'une pièce de viande, il est très important qu'au cours des opérations de découennage, séparation du gras, etc., la nature de chaque milieu soit détectée avec précision et que, par exemple, des morceaux de maigre ne soient pas enlevés avec des morceaux de gras.

Des dispositifs de détection optiques ont déjà été proposés pour permettre de reconnaître le passage du "gras" au "maigre" lors de la découpe d'une pièce de viande ; cependant, ces dispositifs ne donnent pas toujours de très bons résultats, du fait qu'ils ne permettent qu'une faible différenciation entre le gras et le maigre et qu'ils imposent des réajutements des valeurs de seuil de détection. Des exemples de tels dispositifs sont décrits dans FR-A 1 550 169 et US-A 3 224 320.

Des dispositifs décrivant une détection d'épaisseur du gras ou du maigre, fondée sur la variation de résistivité électrique, sont connus de AU-B 482 112 et DE-A 2 107 114.

Des systèmes à caméra permettent également une détection quantitative de la présence de gras et de maigre; cependant, ces systèmes sont compliqués et très coûteux.

Le but de la présente invention est de remédier à ces inconvénients et de fournir un dispositif permettant, d'une façon simple, peu coûteuse et fiable, une détection précise de la nature d'un milieu de viande et du passage d'un milieu dans un autre milieu de nàture différente dans un morceau de viande.

Ce but est atteint par un dispositif de détection de la nature d'un milieu de viande, par mesure de la résistivité du morceau de viande dont on veut déterminer la nature, dispositif qui est formé d'une lame de coupe ou similaire montée sur un support et d'au moins une électrode montée sur le support de la lame de coupe et apte à se déplacer avec celui-ci, cette électrode étant isolée électriquement de la lame de coupe et de son support et étant alimentée par un courant électrique d'intensité déterminée et dans lequel des moyens sont prévus pour mesurer le potentiel de l'électrode par rapport au morceau de viande, celui-ci étant, lui-même, maintenu à un potentiel fixe connu.

Le dispositif conforme à l'invention sera expliqué et d'autres caractéristiques seront mises en évidence à l'aide de la description qui suit en référence au dessin schématique annexé dans lequel:

Figure 1 est une vue en coupe longitudinale d'un exemple de réalisation d'un dispositif de détection ;

Figure 2 est, à échelle réduite, un schéma illustrant le mode de fonctionnement du dispositif de détection de figure 1 ;

Figure 3 est une représentation des courbes I = f(V) dans le gras et dans le maigre.

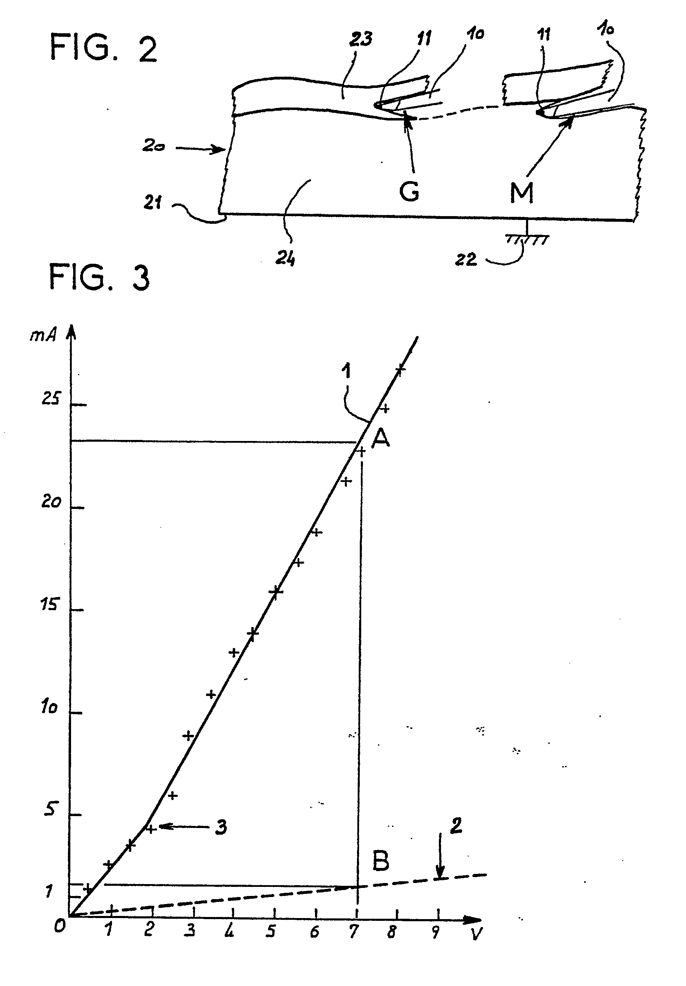

La figure 3 illustre les expériences réalisées dans le cadre de la présente invention sur un "rein brut" de porc, par terme rein brut étant entendu la partie du porc formée par la longe (qui est une partie maigre) et la "bardière" (qui est constituée par la couenne et le gras).

Tout d'abord, l'expérience a consisté à mesurer l'intensité (exprimée en mA) du courant I passant entre deux électrodes appliquées successivement contre une pièce de gras et contre une pièce de maigre, en fonction de la tension V (exprimée en volts) appliquée entre ces deux électrodes.

Les différentes mesures obtenues ont permis de tracer les deux courbes I = f(V) représentées sur la figure 3, la courbe 1 en traits pleins concernant les mesures effectuées dans le milieu maigre, et la courbe 2 en traits pointillés, celles effectuées dans le milieu gras.

Les deux courbes I = f(V) sont des droites, dont la pente correspond à la résistance appliquée entre les deux électrodes, c'est-à-dire à la résistance respective du maigre et du gras.

Les pentes obtenues sont les suivantes :

maigre = 3,8 en mA/V

gras = 0,118 en mA/V

ce qui correspond à des résistances équivalentes respectivement de :

265 ohms pour le maigre

8 500 ohms pour le gras

c'est-à-dire un rapport de plus de 30.

On notera que la courbe 1 présente un léger point d'inflexion 3 qui est dû à un changement de calibre de l'appareil de mesure.

Cette première expérience montre donc que, pour une même tension V, (par exemple 7 V) appliquée entre les deux électrodes, la différence d'intensité (points A et B de la figure 3) mesurée entre le gras et le maigre est très importante (plus de 20 mA) et est donc facilement détectable.

Ds expériences complémentaires ont permis d'obtenir les résultats qui sont regroupés dans les différents tableaux ci-après :

### VARIATION DU COURANT I, A TENSION CONSTANTE EN FONCTION DU MATERIAU DES ELECTRODES

| MATERIAU DES ELECTRODES | I MOYEN DANS LE MAIGRE | I MOYEN DANS LE GRAS | OBSERVATIONS |
|---|---|---|---|
| Laiton étamé | 28 mA | 1 mA | Evolution du courant dans le temps |
| Laiton doré | 16 mA | 0,9 mA | |
| Graphite | 19 mA | 1,4 mA | |
| Aluminium anodisé | 22 mA | 1 mA | Evolution du courant dans le temps |
| Cuivre (désoxydé) | 18 mA | 0,8 mA | Evolution du courant dans le temps |

On notera que, pour chacun des matériaux, les électrodes avaient à peu près la même surface.

Ce tableau montre que le rapport I maigre/I gras est à peu près le même quel que soit le matériau du capteur (électrodes) utilisé, et notamment que le matériau soit inoxydable ou pas.

Bien entendu, il va de soi que les deux électrodes doivent être dans un même matériau pour éviter l'effet de pile.

### RAPPORT I MAIGRE/I GRAS EN FONCTION DE LA TENSION APPLIQUEE

| V-Volt | 10 | 7 | 3 | 2 | 1 | 0,5 |
|---|---|---|---|---|---|---|
| I maigre mA | 20 | 11 | 3,9 | 1,8 | 0,3 | 0,13 |
| I gras mA | 3 | 1,7 | 0,7 | 0,4 | 0,12 | 0,08 |
| I maigre/I gras | 6,7 | 6,5 | 5,6 | 4,5 | 2,5 | 1,6 |

### RAPPORT V GRAS/V MAIGRE EN FOCTION DU COURANT I APPLIQUE

| ImA | 5 | 1 | 0,8 | 0,5 | 0,1 |
|---|---|---|---|---|---|
| V Gras | 20,5 | 7 | 6 | 5 | 0,7 |
| V Maigre | 3,5 | 1,8 | 1,6 | 1,3 | 0,6 |
| V Maigre/V Gras | 5,9 | 3,9 | 3,8 | 3,8 | 1,2 |

Ces tableaux montrent une certaine variation des rapports lorsque la tension ou le courant appliqué sont faibles et montrent donc la nécessité d'effectuer les mesures au-dessus de certaines valeurs limites de tension (7 à 10 V) et de courant (5 mA).

En outre, d'autres mesures ont permis de constater que le courant évolue proportionnellement aux surfaces des électrodes en "contact", à tension constante. Cependant, le courant atteint une valeur seuil de "saturation" à partir d'une surface déterminée, par exemple, dans le cas du maigre, la valeur seuil est de 50 mA pour une tension de 10V, cette valeur étant atteinte pour des surfaces en contact d'environ 5 mm².

Bien entendu, ce seuil de saturation est variable en fonction de la tension appliquée aux bornes des capteurs.

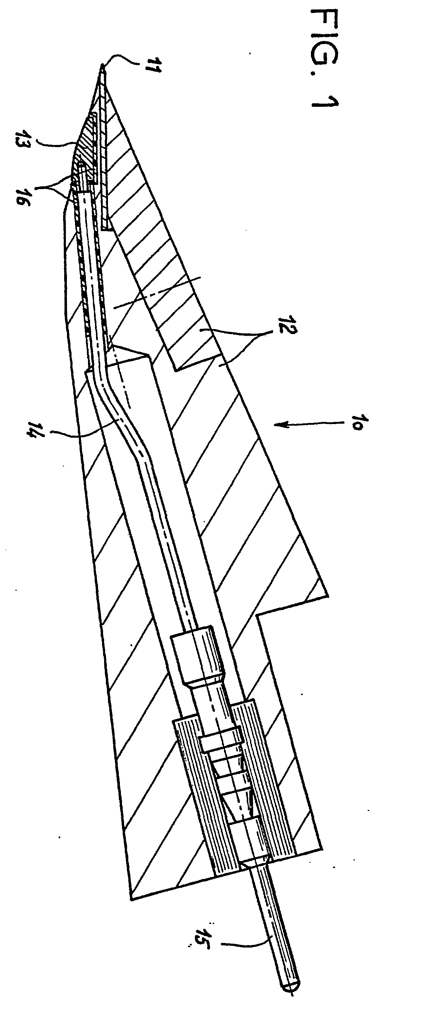

Les figures 1 et 2 montrent un exemple de réalisation d'un dispositif de détection 10 destiné plus particulièrement à l'application du procédé selon l'invention pour la détection de l'interface gras-maigre sur une longe de porc.

Le dispositif 10 de détection selon l'invention est, dans le cas présent, intégré à la lame 11 servant à séparer le gras du maigre et au guide-lame 12 de celle-ci.

Ce dispositif comprend un capteur 13 en acier inoxydable, qui présente une surface lisse et légèrement convexe de façon à ne pas s'encrasser lorsque le capteur est en contact avec le gras ou le maigre.

Ce capteur 13 est fixé sensiblement au même niveau mais légèrement en arrière de la lame 11 afin de ne pas gêner le fonctionnement de celle-ci.

Le capteur 13 est relié par l'intermédiaire d'un fil él;ectrique 14 à un connecteur 15 au moyen duquel une tension ou un courant déterminé peuvent être appliqués au capteur.

L'ensemble capteur 13-fil électrique 14 et connecteur 15 est isolé électriquement du reste du dispositif, à savoir lame 11 + guide-lame 12 par une résine ou similaire 16, cet ensemble lame 11 et guide-lame 12 étant à] un potentiel fixe et notamment à un potentiel nul.

De cette façon, le capteur 13 et l'ensemble lame 11 et guide-lame 12 constituent deux électrodes, dont il suffit de mesurer la différence de potentiel à courant constant, par tout moyen approprié et connu en soi.

La figure 2 illustre un autre mode de mise en oeuvre du procédé dans lequel le morceau de viande (longe de porc 20) est placé sur une plaque métallique 21 reliée à la terre 22 et est donc maintenu à un potentiel nul. Dans ce cas, le dispositif de détection 10 peut être réalisé de la même façon que décrite précédemment, la lame 11 et le guide-lame 12 étant alors à un potentiel quelconque.

Dans cette figure 2, 23 désigne le gras (bardière) tandis que la référence 24 désigne le maigre.

Ainsi que le montre cette figure 2, le dispositif de détection 10 est relié à un dispositif de commande (non représenté sur le dessin), qui réagit en fonction de l'information donnée par ce dispositif de détection,
- de façon à provoquer le déplacement de la lame 11 vers le maigre 24 si le contact avec le gras 23 a été détecté (position G du dispositif),
- et de façon à provoquer le déplacement de la lame 11 vers le gras 23 si le contact avec le maigre 24 a été détecté (position M du dispositif), ce qui permet de ne pas enlever de maigre 24 en même temps que le gras 23.

Dans l'exemple représenté sur les figures 2 et 3, le potentiel au niveau du capteur est, pour un courant constant de 5 mA, d'environ 20 volts pour le gras et d'environ 3 volts pour le maigre.

Le capteur selon l'invention offre l'avantage d'une forte différenciation entre gras et maigre et n'impose pas un réajustement des valeurs seuil de détection comme c'est le cas, par exemple, pour une détection optique.

Il est, en outre, simple à utiliser et à mettre en oeuvre et particulièrement précis.

Bien entendu, ce capteur peut avoir différentes applications, autres que celle décrite ci-avant (détection de l'interface gras-maigre sur des longes de porc) et notamment :
- détection de la présence de maigre sur du gras séparé de la carcasse (bardière par exemple), afin de le récupérer (maigre de catégorie 2) ;
- plus généralement, toute application nécessitant une détection qualitative de la présence de gras et de maigre alternés, ou de couenne et de gras, ou le passage du maigre à l'air ou du gras à l'aponévrose ;
- mesure de l'épaisseur de gras ou de tout autre milieu pour la classification des animaux,

On notera que la mesure de la "résistance" du morceau de viande revient, dans l'exemple décrit ci-avant à mesurer la différence de potentiel à courant constant. C'est, en effet, une telle mesure qui s'est avéréela plus fiable.

Le potentiel mesuré varie d'un milieu à un autre en raison d'un ensemble de phénomènes difficiles à déterminer isolément mais comportant ceux liés à des résistances de contact, des effets de pile, des réactions électrochimiques, etc. Cependant, il va de soi qu'en cas de mesures relatives (passage d'un milieu à un autre, par exemple) l'influence de ces différents phénomènes peut être négligée.

## Revendications

1- Dispositif de détection de la nature d'un milieu de viande, par mesure de la résistivité du morceau (20) de viande dont on veut déterminer la nature, caractérisé en ce qu'il est formé d'une lame de coupe (11) montée sur un support (12) et d'au moins une première électrode (13) montée sur le support (12) et apte à se déplacer avec celui-ci, cette électrode (13) étant isolée électriquement de la lame de coupe (11) et de son support (12) et étant alimentée par un courant électrique d'intensité déterminée et en ce que des moyens sont prévus pour mesurer le potentiel de cette électrode (13) par rapport au morceau (20) de viande, celui-ci étant lui-même maintenu à un potentiel fixe connu.

2- Dispositif selon la revendication 1, caractérisé en ce que le morceau de viande (20) est maintenu à un potentiel nul par l'intermédiaire d'un plaque (22) de mise à la masse sur laquelle il est posé.

3- Dispositif selon la revendication 1, caractérisé en ce qu'une seconde électrode du dispositif est formée par la lame de coupe (11) et ledit support (12), ceux-ci étant reliés à la masse du dispositif.

4- Dispositif selon l'une quelconque des revendications 1 à 3, caractérisé en ce que là première l'électrode (13) est fixée sensiblement au même niveau, mais légèrement en arrière, de la lame de coupe (11).

5- Dispositif selon la revendication 4, caractérisé en ce que la première électrode (13) présente une surface lisse et légèrement convexe.

## Patentansprüche

1. Vorrichtung zur Ermittlung der Natur eines Fleischbereiches, durch Messung des spezifischen elektrischen Widerstandes des Fleischstückes (20), dessen Natur man ermitteln will, dadurch gekennzeichnet, daß sie aus einer Schneidklinge (11), die auf einem Support (12) montiert ist und mindestens einer ersten Elektrode (13) gebildet ist, die auf dem Support (12) montiert ist und darauf ausgelegt ist, sich mit diesem zu bewegen, wobei diese Elektrode (13) elektrisch von der Schneidklinge (11) und dem Support (12) isoliert ist und mit einem elektrischen Strom vorbestimmter Stärke gespeist wird und wobei ferner Mittel vorgesehen sind, um das Potential dieser Elektrode (13) bezüglich des Fleischstückes (20) zu messen, wobei dieses selbst auf einem festen, bekannten Potential gehalten ist.

2. Vorrichtung nach Anspruch 1, dadurch gekennzeichnet, daß das Fleischstück (20) auf einem Nullpotential mittels einer an Masse legenden Platte (22) gehalten ist, auf der es angeordnet ist.

3. Vorrichtung nach Anspruch 1, dadurch gekennzeichnet, daß eine zweite Elektrode der Vorrichtung durch die Schneidklinge (11) und den genannten Support (12) gebildet ist, wobei diese mit der Masse der Vorrichtung verbunden sind.

4. Vorrichtung nach einem der Ansprüche 1 bis 3, dadurch gekennzeichnet, daß die erste Elektrode (13) genau in der gleichen Niveaulage, jedoch leicht dahinter, wie die Schneidklinge (11) befestigt ist.

5. Vorrichtung nach Anspruch 4, dadurch gekennzeichnet, daß die erste Elektrode (13) eine glatte und leicht konvexe Oberfläche aufweist.

**Claims**

1. A device for detecting the nature of the middle of a piece of meat, by measuring the resistivity of the piece (20) of meat, the nature of which is required to be determined, characterised in that it is formed by a cutting blade (11) mounted on a support (12) and at least one first electrode (13) mounted on the support (12) and adapted to move with the latter, this electrode (13) being electrically insulated from the cutting blade (11) and from its support (12) and being supplied with an electrical current of determined strength, and in that means are provided for measuring the potential of this electrode (13) with respect to the piece (20) of meat, the latter itself being maintained at a known fixed potential.

2. A device according to claim 1, characterised in that the piece of meat (20) is held at nil potential by means of an earth plate (22) on which it is placed.

3. A device according to claim 1, characterised in that a second electrode of the device is formed by the cutting blade (11) and said support (12), these latter beinig connected to the earth of the device.

4. A device according to any one of claims 1 to 3, characterised in that the first electrode (13) is fixed substantially at the same level as, but slightly behind, the cutting blade (11).

5. A device according to claim 4, characterised in that the first electrode (13) has a smooth and slightly convex surface.

FIG. 1

FIG. 2

FIG. 3